# EUROPEAN PATENT SPECIFICATION

(11) **EP 4 221 766 B1**
(45) Date of publication and mention of the grant of the patent: **18.09.2024**
(21) Application number: 21798101.8
(22) Date of filing: 27.09.2021
(51) Int. Cl.: A61L 2/10, A61L 2/24

(54) **DEVICE FOR SANITIZING BANKNOTES**
VORRICHTUNG ZUR DESINFEKTION VON BANKNOTEN
DISPOSITIF DE DÉSINFECTION DE BILLETS DE BANQUE

(30) Priority: 02.10.2020 IT 202000005569 U
(43) Date of publication of application: 09.08.2023
(73) Proprietor: SERPILLI, Roberto, 60033 Chiaravalle (AN) (IT)
(72) Inventor: SERPILLI, Roberto, 60033 Chiaravalle (AN) (IT)
(74) Representative: Cerreta, Caterina
(86) International application number: PCT/IB2021/058787
(87) International publication number: WO 2022/070017

(56) References cited:
- WO-A2-2013/068973
- CN-A- 101 352 575
- CN-A- 109 523 688
- US-A1- 2009 301 679
- US-A1- 2014 079 587
- US-B1- 9 117 327

## Description

The present invention relates to a device for sanitizing banknotes.

A particularly deeply felt issue today derives from the need to manipulate banknotes to carry out payments.

Sometimes, in actual fact, cash payment is mandatory, as it is the only payment method allowed; this can happen, for example, when you have to or wish to buy a product or an item in a sales outlet without any devices suitable for receiving payments in other forms than cash, such as the so-called "POS" payment terminals, which allow payments to be made by electronic money, or by credit, debit or prepaid cards.

What has just been said may also apply to self-checkout machines without devices for payment by credit, debit or prepaid cards; in these cases, in order to pay the amount due, it is necessary to insert coins and/or banknotes in special slots provided in the self-checkout machine.

This determines a high risk, for users of physical money (e.g. banknotes and coins), of coming into contact with viruses and/or bacteria and/or spores and/or moulds and/or other pathogens that may be present on the surfaces of the banknotes and/or coins, which could affect the health of the users.

Devices for disinfecting, or sanitizing, banknotes are known, which however have a number of drawbacks and technical limitations mainly linked to the inadequately effective destruction of viruses and bacteria present on banknotes, and also to their structural complexity and, consequently, to their high manufacturing costs.

Some known devices for sanitizing objects, including banknotes, are disclosed in CN101352575A, US2014/079587A1, US9117327B1, WO2013/068973A2, CN109523688A and US2009/301679A1.

The object of the present invention is to solve the prior art issues referred to here above.

Another object of the invention is to provide a device for sanitizing banknotes, which is particularly effective in destroying viruses and/or bacteria and/or spores and/or moulds and/or other pathogens found on the banknotes.

A further object of the invention is to provide a device for sanitizing banknotes, which does not have a complex structure and which is easy and economical to make.

Therefore, a specific object of the present invention is a device for sanitizing banknotes as defined in claim 1 below.

Preferred embodiments are defined in the dependent claims.

Characteristics and advantages will become more obvious from the following description of preferred but not exclusive embodiments illustrated, purely by way of non-limiting example, in the accompanying drawings, wherein:

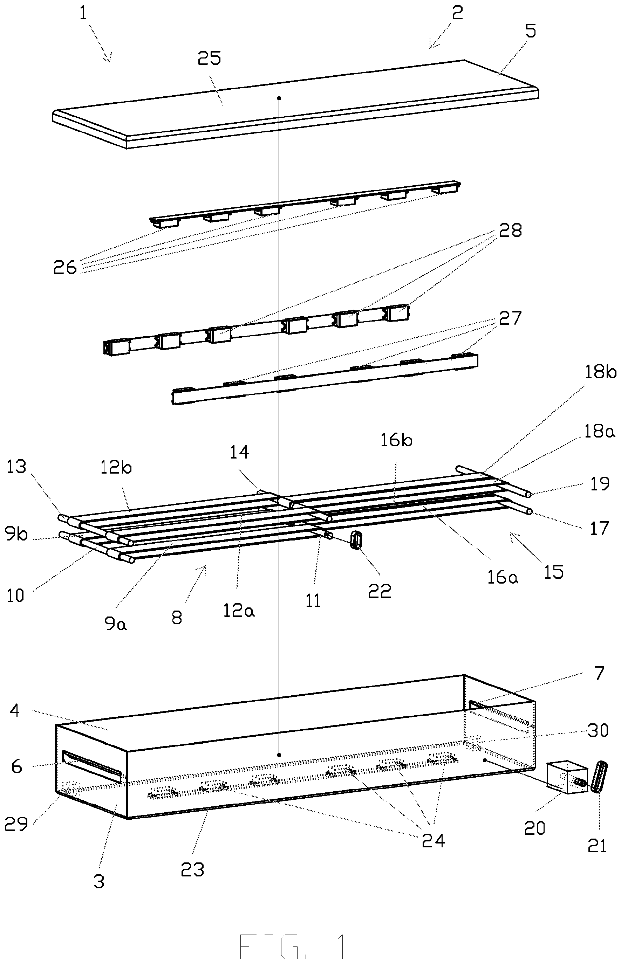

- Figure 1 is an exploded view of a device for sanitizing banknotes according to the present invention;

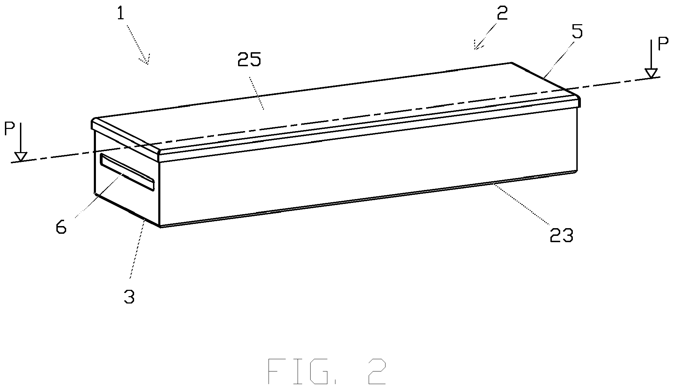

- Figure 2 is an axonometric view of the device shown in Figure 1, in an assembled configuration;
- Figure 3 is a cross-sectional view along the section line P-P indicated in Figure 2, in a first phase of operation of said device, wherein a banknote is represented in a first position;
- Figure 4 is a cross-sectional view taken along the section line A-A indicated in Figure 3;
- Figure 5 is a cross-sectional view along the section line P-P indicated in Figure 2, in a second phase of operation of said device, wherein a banknote is represented in a second position;
- Figure 6 is a cross-sectional view taken along the section line B-B indicated in Figure 5;
- Figure 7 is a cross-sectional view along the section line P-P indicated in Figure 2, in a third phase of operation of said device, wherein a banknote is represented in a third position; and
- Figure 8 is a cross-sectional view taken along the section line C-C indicated in Figure 7.

With reference to the accompanying figures, 1 indicates a device for sanitizing banknotes according to the present invention.

In particular, this device for sanitizing banknotes 1 comprises an external casing 2 consisting of a hollow base 3, wherein a compartment 4 is defined, and a lid 5 namely a cover element that can be pressure-coupled to said base 3 in a removable way.

The aforementioned base 3 has, preferably but not limitedly, a substantially parallelepiped shape.

In two opposite faces of the base 3 there are an inlet opening 6 and an outlet opening 7 to allow for the introduction of banknotes S to be treated and the removal of treated banknotes S, respectively.

Inside the base 3 there is a first conveyor system 8 arranged adjacent to the inlet opening 6 and substantially at the same height as the latter.

The first conveyor system 8 comprises a pair of lower conveyor belts 9a, 9b, with synchronous movement, spaced out and parallel to each other and positioned so as to be parallel to the forward feeding direction D of the banknotes S inside the device for sanitizing banknotes 1.

The conveyor belts of the pair of lower conveyor belts 9a, 9b are wound on a first shaft 10 and a second shaft 11 mounted, parallel to each other, inside the base 3 in a rotatable way around their respective axis and in an orthogonal direction to the aforementioned forward feeding direction D.

The first conveyor system 8 also comprises a pair of upper conveyor belts 12a, 12b, with synchronous movement, in a parallel direction to the forward direction D of the banknotes S and arranged at the same distance existing between the lower conveyor belts 9a, 9b.

The conveyor belts of the above-mentioned pair of upper conveyor belts 12a, 9b are, furthermore, wound on a third shaft 13 and a fourth shaft 14 mounted, parallel to each other, inside the base 3 in a rotatable way around their respective axis and in an orthogonal direction to the forward feeding direction D of the banknotes S inside the device for the sanitizing of banknotes 1.

This pair of upper conveyor belts 12a, 12b is arranged above the aforementioned pair of lower conveyor belts 9a, 9b at a distance such as to allow the introduction, through the inlet opening 6, and the passage of a banknote S in fully extended configuration, in the space between them.

In particular, the two conveyor belts 12a, 12b of the aforementioned pair of upper conveyor belts 12a, 12b are arranged in such positions as to match, respectively, the underlying conveyor belts 9a, 9b of the pair of lower conveyor belts 9a, 9b.

Downstream of the first conveyor system 8, i.e. between this and the outlet opening 7, a second conveyor system 15 is provided.

The second conveyor system 15 also comprises a pair of lower conveyor belts 16a, 16b, with synchronous movement, parallel to each other and oriented in a parallel direction to the aforementioned forward feeding direction D.

The lower conveyor belts 16a, 16b of the second conveyor system 15 are wound on the aforementioned second shaft 11, in the space between the lower conveyor belts 9a, 9b of the first conveyor system 8, and on a fifth shaft 17 mounted, parallel to the second shaft 11, inside the base 3 in a rotatable manner around its axis and directed orthogonally to the forward feeding direction D of the banknotes S inside the device for sanitizing banknotes 1.

Specifically, the first shaft 10, the second shaft 11 and the fifth shaft 17 are located inside the base 3 substantially at the same height; this means that the upper surfaces of the pair of lower conveyor belts 9a, 9b of the first conveyor system 8 and of the pair of lower conveyor belts 16a, 16b of the second conveyor system 15 are substantially coplanar.

The second conveyor system 15 also comprises a pair of upper conveyor belts 18a, 18b, with synchronous movement, in a parallel direction to the forward direction D of the banknotes S and arranged at the same distance existing between the respective lower conveyor belts 16a, 16b underlying them.

Furthermore, the upper conveyor belts 18a, 18b of the second conveyor system 15 are wound on the aforementioned fourth shaft 14, in the space between the upper conveyor belts 12a, 12b of the first conveyor system 8, and on a sixth shaft 19 mounted, parallel to the fourth shaft 14, inside the base 3 in a rotatable manner around its axis and directed orthogonally to the aforementioned forward feeding direction D of the banknotes S.

This pair of upper conveyor belts 18a, 18b of the second conveyor system 15 is arranged above the related pair of lower conveyor belts 16a, 16b at a distance such as to allow the passage of a banknote S, in a fully extended configuration, in the space between them and the removal of said banknote S through the outlet opening 7.

In particular, the upper conveyor belts 18a, 18b of the second conveyor system 15 are arranged in such positions as to match the underlying lower conveyor belts 16a, 16b of the same conveyor system 15, respectively.

The third shaft 13, the fourth shaft 14 and the sixth shaft 19 are located inside the base 3 substantially at the same height; this means that the lower surfaces of the pair of upper conveyor belts 12a, 12b of the first conveyor system 8 and of the pair of upper conveyor belts 18a, 18b of the second conveyor system 15 are substantially coplanar.

To drive the first conveyor system 8 and the second conveyor system 15, a motor 20 is provided, arranged in proximity to the outlet opening 7 and connected, by means of a transmission belt 21, to the fifth shaft 17 to allow the rotation of this shaft on its own axis and therefore the sliding, i.e. the forward feeding according to the aforementioned forward feeding direction D, of the pair of lower conveyor belts 9a, 9b of the first conveyor system 8 and of the pair of lower conveyor belts 16a, 16b of the second conveyor system 15.

To transfer the drive from the two pairs of lower conveyor belts 9a, 9b, 16a, 16b to the two pairs of upper conveyor belts 12a, 12b, 18a, 18b, another transmission belt 22 is provided, mounted on the second shaft 11 and on the fourth shaft 14, so as to transmit rotation from said second shaft 11 to said fourth shaft 14 when the former rotates on its respective axis.

Furthermore, on the inner side of the bottom 23 of the base 3 there is a first plurality of emitters of UVC rays 24, i.e. type C ultraviolet rays, arranged in such a way as to substantially cover the entire longitudinal extension of the base 3, i.e. the extension thereof according to the aforementioned forward direction D, and facing the two pairs of lower conveyor belts 9a, 9b, 16a, 16b so as to directly irradiate the lower exposed surfaces of the banknotes S passing through the first conveyor system 8 and the second conveyor system 15, i.e. the lower surfaces of the same banknotes S not covered by said lower conveyor belts 9a, 9b, 16a, 16b.

Similarly, on the inner side of the upper wall 25 of the lid 5 there is a second plurality of emitters of UVC rays 26, i.e. type C ultraviolet rays, arranged in such a way as to substantially cover the entire longitudinal extension of the lid 5, i.e. the extension thereof according to the aforementioned forward direction D, and facing the two pairs of upper conveyor belts 12a, 12b, 18a, 18b when said lid 5 is applied on to the base 3, so as to directly irradiate the upper exposed surfaces of the banknotes S passing through the first conveyor system 8 and the second conveyor system 15, i.e. the upper surfaces of the same banknotes S not covered by said upper conveyor belts 12a, 12b, 18a, 18b.

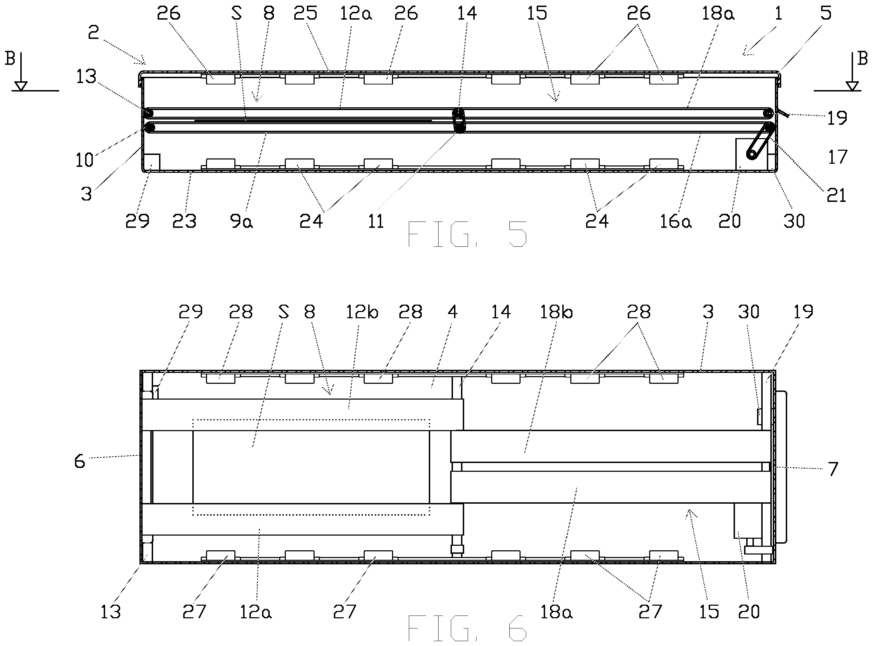

As can be seen from figures 5 and 6, during conveying of the banknote S by means of the first conveyor system 8 described above, the same banknote S is irradiated, from above and below, at its central strip not covered by the pair of upper conveyor belts 12a, 12b and the pair of lower conveyor belts 9a, 9b.

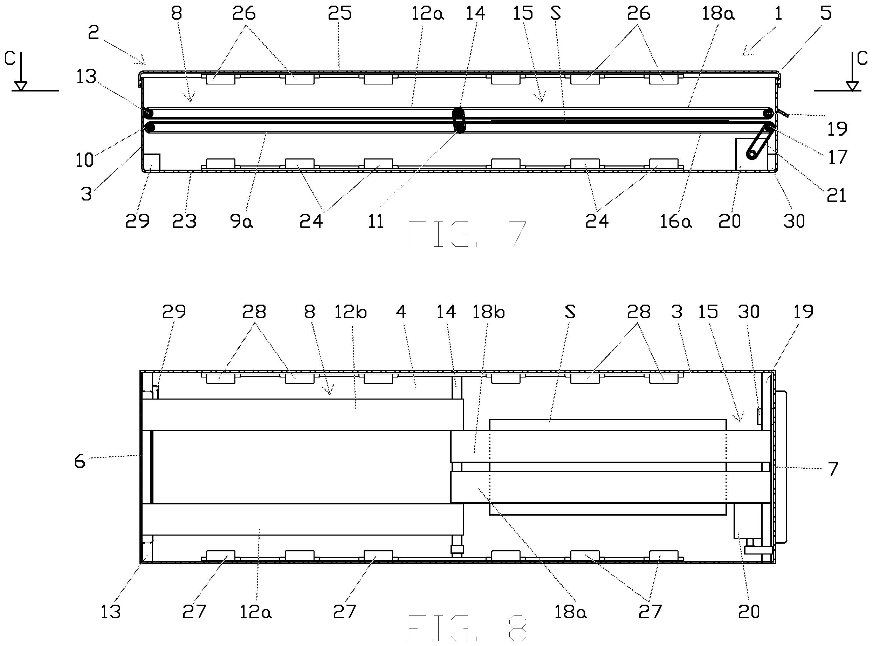

When, on the other hand, the same banknote S is conveyed by the second conveyor system 15, it is irradiated, from above and below, at two lateral longitudinal strips (externally to the pair of upper conveyor belts 18a, 18b and to the pair of lower conveyor belts 16a, 16b of the second conveyor system 15) and at a central longitudinal strip (comprised between the pair of upper conveyor belts 18a, 18b and the pair of lower conveyor belts 16a, 16b of the second conveyor system 15), i.e. at the strips not covered by the pair of upper conveyor belts 18a, 18b and the pair of lower conveyor belts 16a, 16b of the second conveyor system 15 (see Figs. 7 and 8).

Wanting to expand on the concept explained above, the first conveyor system 8 and the second conveyor system 15 are configured in such a way as to allow the conveying of banknotes S from the inlet opening 6 to the outlet opening 7 by making contact to, and therefore covering, different longitudinal areas or strips of the banknotes S so that during the passage of each banknote S through the first conveyor system 8 at least one upper longitudinal strip and the underlying at least one lower longitudinal strip of this banknote S are irradiated (see figs. 5 and 6) and that during its passage through the second conveyor system 15 all the remaining upper and lower longitudinal strips of the banknote S are irradiated (see figs. 7 and 8), so as to obtain total and direct irradiation thereof.

Optionally, a third plurality of UVC ray emitters 27 and a fourth plurality of UVC ray emitters 28 are provided on the inner faces of the two opposite sides of the base 3 not affected by the inlet opening 6 and the outlet opening 7 - respectively, both facing the first conveyor system 8 and the second conveyor system 15 and therefore also the side edges of the banknotes S passing through them.

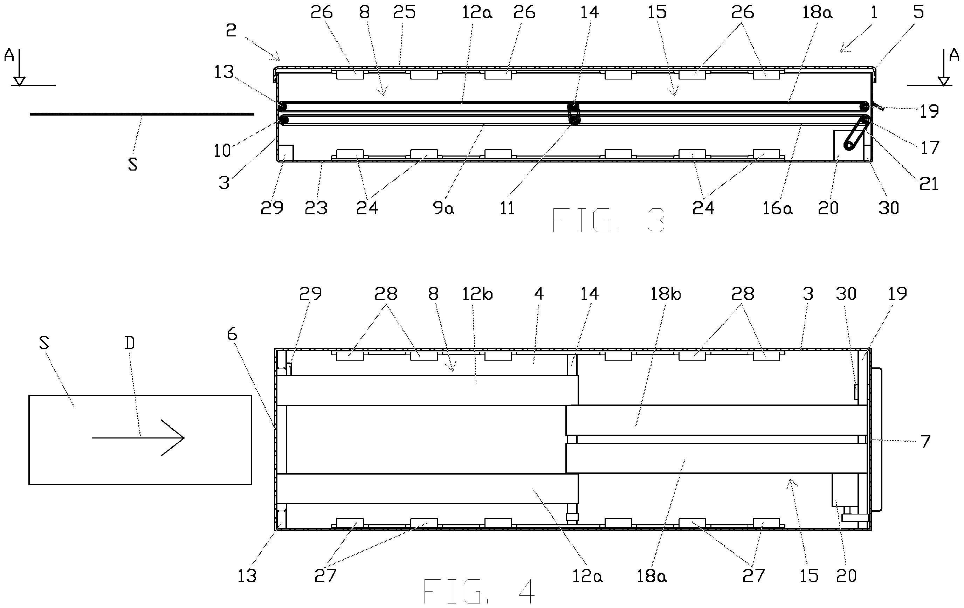

At the inlet opening 6 there is an inlet sensor 29 (for example a presence sensor) suitable for detecting the insertion of the banknotes S through the same inlet opening 6 and operatively connected to the motor 20 and to the aforementioned four pluralities of UVC ray emitters 24, 26, 27, 28, so that when a banknote S is inserted through the inlet opening 6 (see Figs. 3 and 4), this inlet sensor 29 transmits a signal for automatically controlling the activation of the motor 20 and the switching on of the four pluralities of UVC ray emitters 24, 26, 27, 28 to allow direct and complete irradiation of the banknotes S during their transfer from inlet opening 6 to outlet opening 7.

At the inlet opening 7 an inlet sensor 30 is provided instead (for example a presence sensor) suitable for detecting the banknotes S reaching the outlet opening 7 and also operatively connected to the motor 20 and to the aforementioned four pluralities of UVC ray emitters 24, 26, 27, 28, so that the same outlet sensor 30 transmits a signal for automatically controlling the deactivation of the motor 20 and the switching off of the four pluralities of UVC ray emitters 24, 26, 27, 28 when the banknotes S reach the outlet opening 7.

Optionally, a timer can be provided that is operatively connected to the output sensor 30 and operating in such a way as to control, in any case, the switching off of the four pluralities of UVC ray emitters 24, 26, 27, 28 should the output sensor 30 not detect the presence of a banknote within a certain period of time from detection by the inlet sensor 29.

Ozone lamps can be provided in place of or in addition to the first plurality of UVC ray emitters 24 and the second plurality of UVC ray emitters 26, and the possible third plurality of UVC ray emitters 27 and fourth plurality of UVC ray emitters 28.

To improve the conveying of the banknotes S through the first conveyor system 8 and the second conveyor system 15, it is possible to provide a pressing system (not shown in the attached figures) acting on the two pairs of lower conveyor belts 9a, 9b, 16a, 16b and/or on the two pairs of upper conveyor belts 12a, 12b, 18a, 18b, to help the adhesion of the same banknotes S to said conveyor belts and therefore their conveying.

According to a variant of the present invention not shown in the accompanying figures, in replacement of the two pairs of upper conveyor belts 12a, 12b, 18a, 18b and of the relative shafts 13, 14, 19 it is possible to provide a plurality of idle rollers with horizontal rotation axis, possibly kept pushing against the underlying lower conveyor belts 9a, 9b, 16a, 16b by means of a thrust system acting downwards.

In this case, these idle rollers operate in such a way as to push the banknotes S towards the underlying lower conveyor belts 9a, 9b, 16a, 16b, ensuring adhesion to them and supporting their forward feeding, made possible thanks to the driving action of the same lower conveyors belts 9a, 9b, 16a, 16b.

According to another variant of the present invention not shown in the accompanying figures, in replacement of the two pairs of lower conveyor belts 9a, 9b, 16a, 16b and of the related shafts 10, 11, 17 it is possible to provide a plurality of idle rollers with horizontal rotation axis, possibly kept pressurised on the overlying lower conveyor belts 12a, 12b, 18a, 18b by means of a thrust system acting from the bottom upwards.

In this case, these idle rollers operate in such a way as to push the banknotes S towards the overlying upper conveyor belts 12a, 12b, 18a, 18b, ensuring adhesion to them and supporting their forward feeding, made possible thanks to the driving action of the same upper conveyors belts 12a, 12b, 18a, 18b.

According to further embodiments of the present invention not shown in the accompanying figures, the two conveyor systems 8, 15 described above might not be coplanar, but rather arranged one below or above the other, provided that during the entire pathway of the banknote S from the inlet 6 to the outlet 7 of the external envelope 2, both the upper surface and the lower surface of the banknote S are entirely and directly irradiated.

As can be inferred from the above description, the device for sanitizing banknotes 1, according to the present invention, allows complete sanitizing of the banknotes S to be obtained by direct irradiation of all the surfaces of the banknotes.

Furthermore, the structure of the aforementioned device for sanitizing banknotes 1 is not particularly complex and therefore can be implemented in a simple and economical way.

It is understood that the above has been described by way of non-limiting example, so that any construction variations should be considered as falling within the scope of the present technical solution, as long as it stays within the scope of the appended claims.

## Claims

1. Device for sanitizing banknotes (1) comprising:
- a container (2) where are defined an inlet (6) for inserting at least one banknote (S) into the container (2) and an outlet (7) for extracting at least one banknote (S) from the container (2);
- a first conveyor system (8) arranged in the container (2) and configured to convey at least one banknote (S) according to a forward direction (D) from the inlet (6) to a predetermined area between the inlet (6) and the outlet (7) picking said at least one banknote (S) at a first zone of said at least one banknote (S); wherein the first conveyor system (8) comprises at least one first conveyor belt (9a) located adjacent to the inlet (6) and at least one second conveyor belt (12a), or a plurality of revolving elements, arranged above said at least one first conveyor belt (9a) of the first conveyor system (8) such that, in use, said at least one banknote (S) introduced into the inlet (6) is conveyed between said at least one first conveyor belt (9a) and said at least one second conveyor belt (12a), or said plurality of revolving elements, of the first conveyor system (8);
- a second conveyor system (15) arranged in the container (2) and configured to convey said at least one banknote (S) from said predetermined area to the outlet (7) picking said at least one banknote (S) at a second zone of said at least one banknote (S), wherein said second zone is other than said first zone; wherein the second conveyor system (15) comprises at least one first conveyor belt (16a) arranged between said at least one first conveyor belt (9a) of the first conveyor system (8) and the outlet (7) in a horizontally staggered way, according to a direction transversal to the forward direction (D), with respect to said at least one first conveyor belt (9a) of the first conveyor system (8); wherein the second conveyor system (15) comprises at least one second conveyor belt (18a), or a plurality of revolving elements, arranged above said at least one first conveyor belt (16a) of the second conveyor system (15) such that, in use, said at least one banknote (S) is conveyed between said at least one first conveyor belt (16a) and said at least one second conveyor belt (18a), or said plurality of revolving elements, of the second conveyor system (15);
- at least one first UVC ray emitter (24), or at least one first ozone lamp, arranged in the container (2) in such a way as to directly irradiate and sanitize, in use, the entire lower face of at least one banknote (S) conveyed from the inlet (6) to the outlet (7) by the first conveyor system (8) and the second conveyor system (15); and
- at least one second UVC ray emitter (26), or at least one second ozone lamp, arranged in the container (2) in such a way as to directly irradiate and sanitize, in use, the entire upper face of at least one banknote (S) conveyed from the inlet (6) to the outlet (7) by the first conveyor system (8) and the second conveyor system (15).

2. Device for sanitizing banknotes (1) according to claim 1, **characterised in that** it comprises a first sensor (29) configured to detect the introduction of a banknote (S) into the inlet (6) and operatively connected to the first conveyor system (8), to the second conveyor system (15), to said at least one first UVC ray emitter (24), or to said at least one first ozone lamp, and to said at least one second UVC ray emitter (26), or to said at least one second ozone lamp, in such a way that, when a banknote (S) is introduced into the inlet (6), said first sensor (29) emits an electrical signal to order, automatically, the activation of the first conveyor system (8) and of the second conveyor system (15) and the powering on of said at least one first UVC ray emitter (24) or said at least one first ozone lamp, and of said at least one second UVC ray emitter (26) or said at least one second ozone lamp.

3. Device for sanitizing banknotes (1) according to claim 1 or 2, **characterised in that** it comprises a second sensor (30) configured to detect the removal of a banknote (S) from the outlet (7) and operatively connected to the first conveyor system (8), to the second conveyor system (15), to said at least one first UVC ray emitter (24) or to said at least one first ozone lamp, and to said at least one second UVC ray emitter (26) or to said at least one second ozone lamp, in such a way that, when said at least one banknote (S) reaches the outlet (7), said second sensor (30) emits an electrical signal to order, automatically, the deactivation of the first conveyor system (8) and of the second conveyor system (15) and the powering off of said at least one first UVC ray emitter (24) or said at least one first ozone lamp, and of said at least one second UVC ray emitter (26) or said at least one second ozone lamp.

4. Device for sanitizing banknotes (1) according to any one of the preceding claims, **characterized in that** said at least one first conveyor belt (9a) of the first conveyor system (8) and said at least one first conveyor belt (16a) of the second conveyor system (15) are substantially coplanar.

5. Device for sanitising banknotes (1) according to any one of the preceding claims, **characterised in that** it comprises at least one thrusting member or device to push said at least one second conveyor belt (12a), or said plurality of revolving elements, of the first conveyor system (8) against said at least one first conveyor belt (9a) of the first conveyor system (8), or vice versa, and to push said at least one second conveyor belt (18a), or said plurality of revolving items, of the second conveyor system (15) against said at least one first conveyor belt (16a) of the second conveyor system (15), or vice versa.

6. Device for sanitising banknotes (1) according to any one of the preceding claims, **characterised in that** the first conveyor system (8) and/or the second conveyor system (15) each comprise first two conveyor belts (9a, 12a; 16a, 18a) arranged above each other and second two conveyor belts (9b, 12b; 16b, 18b) arranged above each other.

7. Device for sanitising banknotes (1) according to any one of claims 1-3, 5, 6, **characterised in that** the second conveyor system (15) is arranged, with respect to the first conveyor system (8), at a higher or lower height in such a position that when said at least one banknote (S) is conveyed from the inlet (6) to the outlet (7) by the first conveyor system (8) and the second conveyor system (15), both faces of said at least one banknote (S) are directly and completely irradiated by means of said at least one first UVC ray emitter (24), or at least one first ozone lamp, and said at least one second UVC ray emitter (26), or at least a second ozone lamp.

8. Device for sanitising banknotes (1) according to any one of the preceding claims, **characterised in that** it comprises at least one third UVC ray emitter (27), or at least one third ozone lamp, and at least one fourth UVC ray emitter (28), or at least one fourth ozone lamp, arranged in the container (2) opposite each other in such a way as to directly irradiate and sanitize, in use, at least two opposite side edges of said banknote (S) when said banknote (S) is conveyed by the first conveyor system (8) and by the second conveyor system (15).

9. Device for sanitising banknotes (1) according to any one of the preceding claims, **characterised in that** it comprises driving means (20) for driving the first conveyor system (8) and the second conveyor system (15).

## Patentansprüche

1. Vorrichtung zum Desinfizieren von Banknoten (1), umfassend:
- einen Behälter (2), in dem ein Einlauf (6) zum Einführen mindestens einer Banknote (S) in den Behälter (2) und ein Auslauf (7) zum Entnehmen mindestens einer Banknote (S) aus dem Behälter (2) definiert sind;
- ein erstes Fördersystem (8), das in dem Behälter (2) angeordnet und dazu ausgelegt ist, mindestens eine Banknote (S) gemäß einer Vorwärtsrichtung (D) von dem Einlauf (6) in einen vorgegebenen Bereich zwischen dem Einlauf (6) und dem Auslauf (7) zu fördern, indem es die mindestens eine Banknote (S) in einer ersten Zone der mindestens einen Banknote (S) aufnimmt; wobei das erste Fördersystem (8) mindestens ein erstes Förderband (9a), das benachbart zu dem Einlauf (6) liegt, und mindestens ein zweites Förderband (12a) oder eine Vielzahl von umlaufenden Elementen umfasst, die über dem mindestens einen ersten Förderband (9a) des ersten Fördersystems (8) angeordnet ist, derart, dass im Gebrauch die mindestens eine in den Einlauf (6) eingeführte Banknote (S) zwischen dem mindestens einen ersten Förderband (9a) und dem mindestens einen zweiten Förderband (12a) oder der Vielzahl von umlaufenden Elementen des ersten Fördersystems (8) befördert wird;
- ein zweites Fördersystem (15), das in dem Behälter (2) angeordnet ist und dazu ausgelegt ist, die mindestens eine Banknote (S) von dem vorgegebenen Bereich zu dem Auslauf (7) zu befördern, indem sie die mindestens eine Banknote (S) in einer zweiten Zone der mindestens einen Banknote (S) aufnimmt, wobei die zweite Zone von der ersten Zone verschieden ist; wobei das zweite Fördersystem (15) mindestens ein erstes Förderband (16a), das gemäß einer Richtung, die quer zur Vorwärtsrichtung (D) verläuft, in Bezug auf das mindestens eine erste Förderband (9a) des ersten Fördersystems (8), zwischen dem mindestens einen ersten Förderband (9a) des ersten Fördersystems (8) und dem Auslauf (7) horizontal versetzt angeordnet ist; wobei das zweite Fördersystem (15) mindestens ein zweites Förderband (18a) oder eine Vielzahl von umlaufenden Elementen umfasst, die über dem mindestens einen ersten Förderband (16a) des zweiten Fördersystems (15) angeordnet ist, derart, dass im Gebrauch die mindestens eine Banknote (S) zwischen dem mindestens einen ersten Förderband (16a) und dem mindestens einen zweiten Förderband (18a) oder der Vielzahl von umlaufenden Elementen des zweiten Fördersystems (15) befördert wird;
- mindestens einen ersten UVC-Strahler (24) oder mindestens eine erste Ozonlampe, der bzw. die derart im Behälter (2) angeordnet ist, dass im Gebrauch die gesamte untere Seite von mindestens einer Banknote (S), die durch das erste Fördersystem (8) und das zweite Fördersystem (15) vom Einlauf (6) zum Auslauf (7) befördert wird, direkt bestrahlt und desinfiziert wird; und
- mindestens einen zweiten UVC-Strahler (26) oder mindestens eine zweite Ozonlampe, der bzw. die derart im Behälter (2) angeordnet ist, dass im Gebrauch die gesamte obere Seite von mindestens einer Banknote (S), die durch das erste Fördersystem (8) und das zweite Fördersystem (15) vom Einlauf (6) zum Auslauf (7) befördert wird, direkt bestrahlt und desinfiziert wird.

2. Vorrichtung zum Desinfizieren von Banknoten (1) nach Anspruch 1, **dadurch gekennzeichnet, dass** sie einen ersten Sensor (29) umfasst, der dazu ausgelegt ist, das Einführen einer Banknote (S) in den Einlauf (6) zu erkennen, und funktional derart mit dem ersten Fördersystem (8), dem zweiten Fördersystem (15), dem mindestens einen ersten UVC-Strahler (24) oder der mindestens einen ersten Ozonlampe und dem mindestens einen zweiten UVC-Strahler (26) oder der mindestens einen zweiten Ozonlampe verbunden ist, dass der erste Sensor (29), wenn eine Banknote (S) in den Einlauf (6) eingeführt wird, ein elektrisches Signal aussendet, um automatisch die Aktivierung des ersten Fördersystems (8) und des zweiten Fördersystems (15) und das Einschalten des mindestens einen ersten UVC-Strahlers (24) oder der mindestens einen ersten Ozonlampe und des mindestens einen zweiten UVC-Strahlers (26) oder der mindestens einen zweiten Ozonlampe anzuweisen.

3. Vorrichtung zum Desinfizieren von Banknoten (1) nach Anspruch 1 oder 2, **dadurch gekennzeichnet, dass** sie einen zweiten Sensor (30) umfasst, der dazu ausgelegt ist, das Entfernen einer Banknote (S) aus dem Auslauf (7) zu erkennen, und funktional derart mit dem ersten Fördersystem (8), dem zweiten Fördersystem (15), dem mindestens einen ersten UVC-Strahler (24) oder der mindestens einen ersten Ozonlampe und dem mindestens einen zweiten UVC-Strahler (26) oder der mindestens einen zweiten Ozonlampe verbunden ist, dass, wenn die mindestens eine Banknote (S) den Auslauf (7) erreicht, der zweite Sensor (30) ein elektrisches Signal aussendet, um automatisch die Deaktivierung des ersten Fördersystem (8) und des zweiten Fördersystems (15) und die Deaktivierung des mindestens einen ersten UVC-Strahlers (24) oder der mindestens einen ersten Ozonlampe und des mindestens einen zweiten UVC-Strahlers (26) oder der mindestens einer zweiten Ozonlampe anzuweisen.

4. Vorrichtung zum Desinfizieren von Banknoten (1) nach einem der vorstehenden Ansprüche, **dadurch gekennzeichnet, dass** mindestens ein erstes Förderband (9a) des ersten Fördersystems (8) und mindestens ein erstes Förderband (16a) des zweiten Fördersystems (15) im Wesentlichen komplanar sind.

5. Vorrichtung zum Desinfizieren von Banknoten (1) nach einem der vorstehenden Ansprüche, **dadurch gekennzeichnet, dass** sie mindestens ein Schuborgan oder eine Schubvorrichtung umfasst, um das mindestens eine zweite Förderband (12a) oder die Vielzahl von umlaufenden Elementen des ersten Fördersystems (8) gegen das mindestens eine erste Förderband (9a) des ersten Fördersystems (8) zu drücken oder umgekehrt, und das mindestens eine zweite Förderband (18a) oder die Vielzahl von umlaufenden Elementen des zweiten Fördersystems (15) gegen das mindestens eine erste Förderband (16a) des zweiten Fördersystems (15) zu drücken oder umgekehrt.

6. Vorrichtung zum Desinfizieren von Banknoten (1) nach einem der vorstehenden Ansprüche, **dadurch gekennzeichnet, dass** das erste Fördersystem (8) und/oder das zweite Fördersystem (15) jeweils erste zwei übereinander angeordnete Förderbänder (9a, 12a; 16a, 18a) und zweite zwei übereinander angeordnete Förderbänder (9b, 12b; 16b, 18b) umfassen.

7. Vorrichtung zum Desinfizieren von Banknoten (1) nach einem der Ansprüche 1 bis 3, 5, 6, **dadurch gekennzeichnet, dass** das zweite Fördersystem (15) in Bezug auf das erste Fördersystem (8) in einer höheren oder niedrigeren Höhe angeordnet ist, derart, dass, wenn die mindestens eine Banknote (S) durch das erste Fördersystem (8) und das zweite Fördersystem (15) vom Einlauf (6) zum Auslauf (7) befördert wird, beide Seiten der mindestens einen Banknote (S) direkt und vollständig mit dem mindestens einen ersten UVC-Strahler (24) oder der mindestens einen ersten Ozonlampe und dem mindestens einen zweiten UVC-Strahler (26) oder der mindestens einen zweiten Ozonlampe bestrahlt werden.

8. Vorrichtung zum Desinfizieren von Banknoten (1) nach einem der vorstehenden Ansprüche, **dadurch gekennzeichnet, dass** sie mindestens einen dritten UVC-Strahler (27) oder mindestens eine dritte Ozonlampe und mindestens einen vierten UVC-Strahler (28) oder mindestens eine vierte Ozonlampe umfasst, die in dem Behälter (2) einander gegenüberliegend angeordnet sind, derart, dass im Gebrauch mindestens zwei gegenüberliegende Seitenkanten der Banknote (S) direkt bestrahlt und desinfiziert werden, wenn die Banknote (S) durch das erste Fördersystem (8) und das zweite Fördersystem (15) befördert wird.

9. Vorrichtung zum Desinfizieren von Banknoten (1) nach einem der vorstehenden Ansprüche, **dadurch gekennzeichnet, dass** sie eine Antriebseinrichtung (20) zum Antreiben des ersten Fördersystems (8) und des zweiten Fördersystems (15) umfasst.

## Revendications

1. Dispositif de désinfection de billets de banque (1) comprenant :
- un contenant (2) où sont définies une entrée (6) pour insérer au moins un billet de banque (S) dans le contenant (2) et une sortie (7) pour extraire au moins un billet de banque (S) du contenant (2) ;
- un premier système transporteur (8) disposé dans le contenant (2) et configuré pour transporter au moins un billet de banque (S) selon une direction d'avance (D) de l'entrée (6) à une zone prédéterminée entre l'entrée (6) et la sortie (7) en prélevant ledit au moins un billet de banque (S) au niveau d'une première zone dudit au moins un billet de banque (S) ; où le premier système transporteur (8) comprend au moins une première bande transporteuse (9a) située adjacente à l'entrée (6) et au moins une deuxième bande transporteuse (12a), ou une pluralité d'éléments rotatifs, disposée au-dessus de ladite au moins une première bande transporteuse (9a) du premier système transporteur (8) de manière à ce que, en cours d'utilisation, ledit au moins un billet de banque (S) introduit dans l'entrée (6) soit transporté entre ladite au moins une première bande transporteuse (9a) et ladite au moins une deuxième bande transporteuse (12a), ou ladite pluralité d'éléments rotatifs, du premier système transporteur (8) ;
- un deuxième système transporteur (15) disposé dans le contenant (2) et configuré pour transporter ledit au moins un billet de banque (S) de ladite zone prédéterminée à la sortie (7) en prélevant ledit au moins un billet de banque (S) au niveau d'une deuxième zone dudit au moins un billet de banque (S), où ladite deuxième zone est autre que ladite première zone ; où le deuxième système transporteur (15) comprend au moins une première bande transporteuse (16a) disposée entre ladite au moins une première bande transporteuse (9a) du premier système transporteur (8) et la sortie (7) de façon horizontalement décalée, selon une direction transversale à la direction d'avance (D), par rapport à ladite au moins une première bande transporteuse (9a) du premier système transporteur (8) ; où le deuxième système transporteur (15) comprend au moins une deuxième bande transporteuse (18a), ou une pluralité d'éléments rotatifs, disposée au-dessus de ladite au moins une première bande transporteuse (16a) du deuxième système transporteur (15) de manière à ce que, en cours d'utilisation, ledit au moins un billet de banque (S) soit transporté entre ladite au moins une première bande transporteuse (16a) et ladite au moins une deuxième bande transporteuse (18a), ou ladite pluralité d'éléments rotatifs, du deuxième système transporteur (15) ;
- au moins un premier émetteur de rayons UVC (24), ou au moins une première lampe à ozone, disposé dans le contenant (2) de manière à irradier directement et à désinfecter, en cours d'utilisation, toute la face inférieure d'au moins un billet de banque (S) transporté de l'entrée (6) à la sortie (7) par le premier système transporteur (8) et le deuxième système transporteur (15) ; et
- au moins un deuxième émetteur de rayons UVC (26), ou au moins une deuxième lampe à ozone, disposé dans le contenant (2) de manière à irradier directement et à désinfecter, en cours d'utilisation, toute la face supérieure d'au moins un billet de banque (S) transporté de l'entrée (6) à la sortie (7) par le premier système transporteur (8) et le deuxième système transporteur (15).

2. Dispositif de désinfection de billets de banque (1) selon la revendication 1, **caractérisé en ce qu'**il comprend un premier capteur (29) configuré pour détecter l'introduction d'un billet de banque (S) dans l'entrée (6) et opérationnellement relié au premier système transporteur (8), au deuxième système transporteur (15), audit au moins un premier émetteur de rayons UVC (24), ou à ladite au moins une première lampe à ozone, et audit au moins un deuxième émetteur de rayons UVC (26), ou à ladite au moins une deuxième lampe à ozone, de manière à ce que, lorsqu'un billet de banque (S) est introduit dans l'entrée (6), ledit premier capteur (29) émette un signal électrique pour commander, automatiquement, l'activation du premier système transporteur (8) et du deuxième système transporteur (15) et la mise sous tension dudit au moins un premier émetteur de rayons UVC (24) ou de ladite au moins une première lampe à ozone, et dudit au moins un deuxième émetteur de rayons UVC (26) ou de ladite au moins une deuxième lampe à ozone.

3. Dispositif de désinfection de billets de banque (1) selon la revendication 1 ou 2, **caractérisé en ce qu'**il comprend un deuxième capteur (30) configuré pour détecter le retrait d'un billet de banque (S) de la sortie (7) et opérationnellement relié au premier système transporteur (8), au deuxième système transporteur (15), audit au moins un premier émetteur de rayons UVC (24) ou à ladite au moins une première lampe à ozone, et audit au moins un deuxième émetteur de rayons UVC (26) ou à ladite au moins une deuxième lampe à ozone, de manière à ce que, lorsque ledit au moins un billet de banque (S) atteint la sortie (7), ledit deuxième capteur (30) émette un signal électrique pour commander, automatiquement, la désactivation du premier système transporteur (8) et du deuxième système transporteur (15) et la mise hors tension dudit au moins un premier émetteur de rayons UVC (24) ou de ladite au moins une première lampe à ozone, et dudit au moins un deuxième émetteur de rayons UVC (26) ou de ladite au moins une deuxième lampe à ozone.

4. Dispositif de désinfection de billets de banque (1) selon l'une quelconque des revendications précédentes, **caractérisé en ce que** ladite au moins une première bande transporteuse (9a) du premier système transporteur (8) et ladite au moins une première bande transporteuse (16a) du deuxième système transporteur (15) sont essentiellement coplanaires.

5. Dispositif de désinfection de billets de banque (1) selon l'une quelconque des revendications précédentes, **caractérisé en ce qu'**il comprend au moins un organe ou dispositif de poussée pour pousser ladite au moins une deuxième bande transporteuse (12a), ou ladite pluralité d'éléments rotatifs, du premier système transporteur (8) contre ladite au moins une première bande transporteuse (9a) du premier système transporteur (8), ou inversement, et pour pousser ladite au moins une deuxième bande transporteuse (18a), ou ladite pluralité d'éléments rotatifs, du deuxième système transporteur (15) contre ladite au moins une première bande transporteuse (16a) du deuxième système transporteur (15), ou inversement.

6. Dispositif de désinfection de billets de banque (1) selon l'une quelconque des revendications précédentes, **caractérisé en ce que** le premier système transporteur (8) et/ou le deuxième système transporteur (15) comprennent chacun deux premières bandes transporteuses (9a, 12a ; 16a, 18a) disposées l'une au-dessus de l'autre et deux deuxièmes bandes transporteuses (9b, 12b ; 16b, 18b) disposées l'une au-dessus de l'autre.

7. Dispositif de désinfection de billets de banque (1) selon l'une quelconque des revendications 1-3, 5, 6, **caractérisé en ce que** le deuxième système transporteur (15) est disposé, par rapport au premier système transporteur (8), à une hauteur supérieure ou inférieure dans une position telle que, lorsque ledit au moins un billet de banque (S) est transporté de l'entrée (6) à la sortie (7) par le premier système transporteur (8) et le deuxième système transporteur (15), les deux faces dudit au moins un billet de banque (S) sont directement et complètement irradiées au moyen dudit au moins un premier émetteur de rayons UVC (24), ou de ladite au moins une première lampe à ozone, et dudit au moins un deuxième émetteur de rayons UVC (26), ou de ladite au moins une deuxième lampe à ozone.

8. Dispositif de désinfection de billets de banque (1) selon l'une quelconque des revendications précédentes, **caractérisé en ce qu'**il comprend au moins un troisième émetteur de rayons UVC (27), ou au moins une troisième lampe à ozone, et au moins un quatrième émetteur de rayons UVC (28), ou au moins une quatrième lampe à ozone, disposés dans le contenant (2) à l'opposé l'un de l'autre de manière à irradier directement et à désinfecter, en cours d'utilisation, au moins deux bords latéraux opposés dudit billet de banque (S) lorsque ledit billet de banque (S) est transporté par le premier système transporteur (8) et par le deuxième système transporteur (15).

9. Dispositif de désinfection de billets de banque (1) selon l'une quelconque des revendications précédentes, **caractérisé en ce qu'**il comprend des moyens d'entraînement (20) pour entraîner le premier système transporteur (8) et le deuxième système transporteur (15).
